# EUROPEAN PATENT APPLICATION

(11) **EP 1 393 686 A1**
(43) Date of publication of application: **03.03.2004**
(21) Application number: 03254995.8
(22) Date of filing: 12.08.2003
(51) Int. Cl.: A61B 17/435, F28D 7/12

(54) **Oocyte and embryo handling apparatus regulating the temperature within a tube**

(30) Priority: 30.08.2002 GB 0220146; 11.06.2003 GB 0313392
(71) Applicant: Smiths Group plc, London, NW11 8DS (GB)
(72) Inventor: Bateman, Timothy, Dymchurch, Kent TN29 0QD (GB); Nash, John Edward, Hythe, Kent CT21 4QU (GB)
(74) Representative: Flint, Jonathan McNeill

(57) **Abstract**

Oocyte extraction apparatus or embryo replacement apparatus has a flexible tube (6,7,100) along which the oocyte or embryo passes. The tube (6,7,100) extends along an outer jacket (20,30,113), which maintains the temperature within the tube. The jacket may be thermally insulating or it may be heated such as with warmed liquid.

## Description

This invention relates to oocyte and embryo handling apparatus of the kind for use in the handling an oocyte or embryo including a tube adapted for use in the handling of the oocyte or embryo.

Conventionally, an oocyte is extracted from a follicle using a needle to which suction is applied so that the oocyte is aspirated into a test tube or other receptacle. Often a flushing liquid is supplied along a secondary lumen of the needle to assist removal. Once the oocyte has been extracted, it is transferred to a suitable receptacle

One problem with the apparatus used in this technique is that the temperature of the liquid used to help flush out the oocytes from the patient can fall below the ideal level, especially if the procedure is delayed. Also, the temperature of the oocyte can fall appreciably during its passage along the needle, its associated tubing and into the test tube. Similar problems exist when handling embryos.

It is an object of the present invention to provide alternative apparatus for use in handling an oocyte or embryo.

According to the present invention there is provided apparatus of the above-specified kind, characterised in that the apparatus includes an elongate jacket extending along the tube, and that the jacket is arranged to maintain the temperature within the tube.

The jacket is preferably flexible and may be thermally insulating. Alternatively, the jacket may be heated, such as by a warmed fluid. The jacket may include a central bore in which the tube is received and two outer channels along which the warmed fluid is supplied. The jacket preferably has an inlet and outlet for flow of the fluid into and out of the jacket, the inlet and outlet being located at the same end of the jacket. The tube may be adapted for flow of flushing liquid along it or for passage of an oocyte or embryo. The apparatus may include a dual-lumen oocyte recovery needle, the tube being connected to a flushing lumen of the needle, an aspiration tube being connected with the second lumen of the needle, and a second elongate jacket extending along the aspiration tube to maintain the temperature of an oocyte flowing along the aspiration tube.

Apparatus according to the present invention will now be described, by way of example, with reference to the accompanying drawings, in which:
- Figure 1: is a view of the apparatus;
- Figure 2: is a partly cut-away perspective view of a syringe for flushing liquid;
- Figure 3: is a partly cut-away view of a part of a modified form of the apparatus; and
- Figure 4: is a perspective view of alternative apparatus.

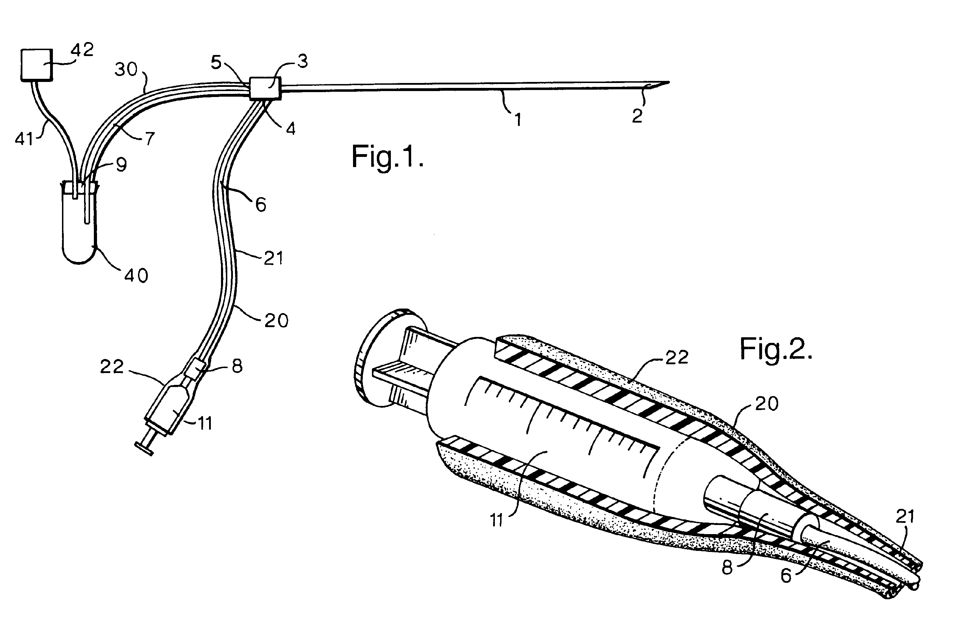

With reference first to Figure 1 the apparatus includes a conventional dual-lumen oocyte recovery needle 1 having a forward, patient end tip 2 and a rear end hub 3. The two lumens within the needle are respectively for supplying flushing liquid and for passage in the opposite direction of aspirated materials including oocytes and follicle fluid. The flushing and aspiration lumens in the needle connect with respective ports 4 and 5 on the hub 3. Attached with the flush and suction ports 4 and 5 are respective tubes 6 and 7. The flushing tube 6 is terminated by a connector 8. The aspiration tube 7 extends through a bung 9 fitted into the neck of a test tube 40. A second tube 41 extends through the bung 9 into the test tube 40 at one end and is connected at its other end with a vacuum pump 42.

The apparatus further includes a syringe 11 connected to the flushing tube 6 and containing a suitable flushing fluid warmed to body temperature of about 37°C.

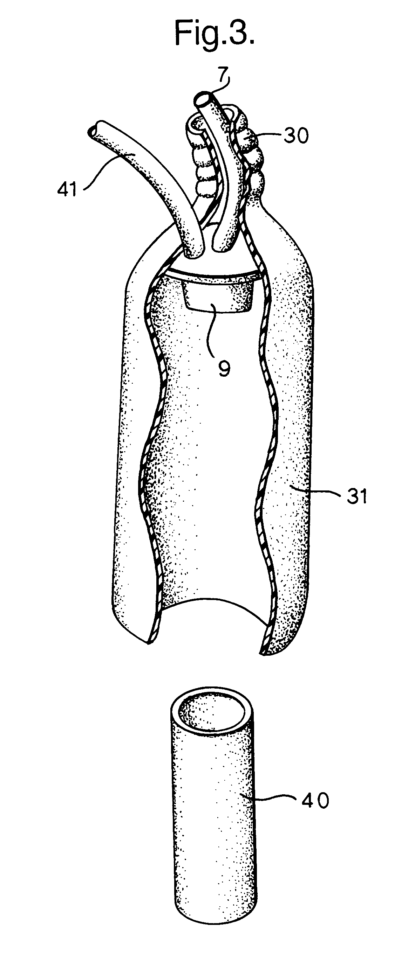

The apparatus is completed by two thermally-insulating jackets 20 and 30 made of a thermally-insulating, flexible material such as a foamed plastics. The jacket 20 has a relatively narrow portion 21 along the major part of its length, which encloses the entire length of the tube 6 as a loose fit and has an enlarged rear end 22 enclosing the barrel of the syringe 11 as a close sliding fit. The other jacket 30 extends along the aspiration tube 7 from the hub 3 to the bung 9. The jacket 30 terminates at the bung 9 although, in an alternative arrangement shown in Figure 3, it could have an enlarged portion 31 at its rear end that fits over the bung 9 and test tube 40.

In operation, the syringe 11 is filled with a flushing liquid previously warmed to body temperature. The nose of the syringe 11 is fitted onto the connector 8 at the end of the flush tube 6 and the enlarged region 22 of the jacket 20 is slid rearwardly to cover the barrel of the syringe. The needle 1 is then used in the usual way to remove oocytes from the patient, turning on the vacuum pump 42 to suck oocytes into the test tube 40, which would normally be in a warmed holder.

The insulating jackets of the present invention help maintain the temperature of the flushing liquid before use and of the oocyte after removal from the patient. The insulating jackets are low cost so they can be disposed of after a single use.

Although the insulating jackets described above are removable from the syringe and tubes and can be used with conventional oocyte recovery sets, they could, instead, be fixed with the syringe or tube. The jackets could be moulded about the apparatus.

The jackets described above maintain the temperature of the syringe and tubes by being thermally insulating. They could, however, maintain the temperature in other ways. For example, the jackets could contain a volume of a previously heated substance such as a wax or gel that maintains the desired temperature by changing from a liquid to a solid at around body temperature. Alternatively, the jackets could include an electrical heating element, which could be powered by an internal battery or a remote power source.

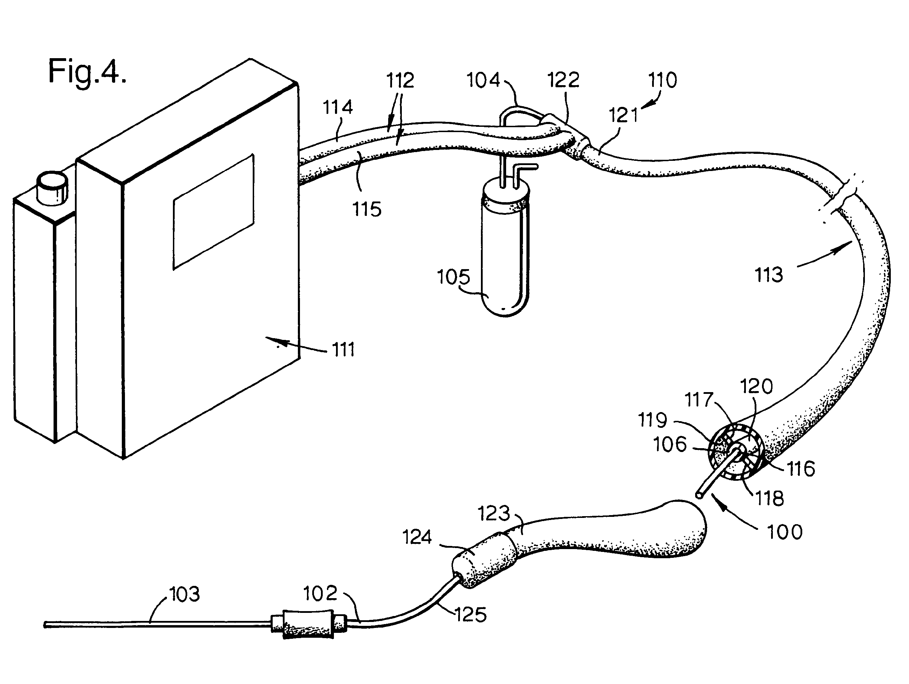

Figure 4 illustrates apparatus having a jacket heated by warmed fluid circulated through the jacket.

The apparatus shown in Figure 4 has a conventional oocyte recovery tube or catheter 100 connected at its forward end 102 to a conventional oocyte recovery needle 103. At its rear end 104, the tube 100 is connected to a collection test tube 105 to which suction is applied so that the recovered oocytes are collected in the tube, in the usual way.

The apparatus has warming arrangement 110 comprising an electrically-heated fluid warmer 111, such as of the kind sold under the trade mark HOTLINE (a Registered Trade Mark of Level 1, Inc) by Level 1, Inc of Rockland, Massachusetts, USA. The fluid warmer 111 provides a recirculating flow of water warmed to about 39°C. The warmer 111 is connected by a dual-bore tubing 112 to a heat exchanger 113. The tubing 112 has two bores 114 and 115, one for the warmed water flowing away from the warmer 111 and the other for the returned water flowing back to the warmer for reheating.

The heat exchanger 113 is preferably a modified version of that used with the HOTLINE warmer for warming infusion fluid supplied to a patient. The heat exchanger 113 is described in detail in US 5063994 and in US 5097898. The heat exchanger 113 is extruded from a plastics material to form a flexible, elongate, tubular jacket of circular external section. The exchanger 113 has three bores extending in parallel along the length of the exchanger. A central bore 116 is provided by a tubular portion 106 of circular section supported by two radially-extending webs 117 and 118. The webs 117 and 118 bisect an outer concentric passage around the central bore 116 into two outer channels 119 and 120 each having a C shape section. The central bore 116 opens axially at the rear end 121 of the exchanger 113 through as an end cap 122. The outer channels 119 and 120 communicate via the cap 122 with respective ones of the bores 114 and 115 of the tubing 112 and hence communicate with the fluid warmer 111. The forward end 123 of the exchanger 113 similarly has an end cap 124 within which the two outer channels 119 and 120 communicate with one another so that water supplied forwardly along one channel can flow back rearwardly along the other channel. The central bore 116 opens axially through the forward end cap 124.

The oocyte recovery tube 100 extends through the central bore 116 of the heat exchanger 113 as a close sliding fit to ensure that there is a good thermal contact between the tube and the tubular portion 106, thereby promoting efficient heat transfer to the tube. The bore 116 or tube 100 may be coated with a lubricant such as a gel to ease insertion of the tube in the bore and to improve thermal contact. The heat exchanger 113 extends along most of the length of the recovery tube 100 apart from a short section 125 at its forward end, which is exposed to facilitate manipulation, although the flexible nature of the heat exchanger does allow the remainder of the tube to be easily manoeuvred.

In an alternative arrangement, the recovery tube would not project from the heat exchanger and, instead, the hub of the needle would be combined with the end cap of the heat exchanger. Such an arrangement has the advantage of enabling the entire length of the oocyte recovery tubing to be warmed. In order to ensure that the needle could still be manipulated freely it would be preferable for the material of the heat exchanger to be selected for maximum flexibility.

In use, the fluid warmer 111 supplies warmed water at about body temperature to the rear end 121 of the exchanger 113, which flows forwardly along one channel 119 and then rearwardly along the other channel 120. This ensures that the oocyte recovery tube 100 is maintained very close to body temperature so as to maintain the recovered oocytes close to their ideal temperature during recovery.

Although alternative forms of heat exchanger could be used, the arrangement described, where fluid is supplied to and returned from the exchanger at its rear end, has several advantages. First, it keeps the forward end of the exchanger free of any additional tubing so that the surgical site is kept uncluttered and so that there is less impediment to manipulation of the forward end of the tube and exchanger. Second, the drop in temperature of the heated water flowing forwardly along the length of the exchanger is in the opposite sense to that of the water flowing rearwardly. This has the effect of reducing the overall temperature gradient along the length of the exchanger, which minimizes the thermal shock to which the oocytes are exposed.

The oocyte recovery tube could be inserted in the heat exchanger by the user and removed after use so that the heat exchanger can be reused. There is no risk of contamination because there is no direct contact with the heat exchanger itself.

The invention is not confined to oocyte recovery apparatus but could also be used in apparatus for use in handling embryos.

## Claims

1. Apparatus for use in the handling an oocyte or embryo including a tube (6, 7, 100) adapted for use in the handling of the oocyte or embryo, **characterised in that** the apparatus includes an elongate jacket (20, 30, 113) extending along the tube, and that the jacket is arranged to maintain the temperature within the tube.

2. Apparatus according to Claim 1, **characterised in that** the jacket (20, 30, 113) is flexible.

3. Apparatus according to Claim 1 or 2, **characterised in that** the jacket (20, 30) is thermally insulating.

4. Apparatus according to Claim 1 or 2, **characterised in that** the jacket (113) is heated.

5. Apparatus according to Claim 4, **characterised in that** the jacket (113) is heated by warmed fluid.

6. Apparatus according Claim 5, **characterised in that** the jacket (113) has a central bore (116) in which the tube (100) is received and two outer channels (119, 120) along which the warmed fluid is supplied.

7. Apparatus according to Claim 5 or 6, **characterised in that** the jacket (113) has an inlet (114) and outlet (115) for flow of the fluid into and out of the jacket, and that the inlet and outlet are located at the same end of the jacket.

8. Apparatus according to any one of the preceding claims, **characterised in that** the tube (6) is adapted for flow of flushing fluid along it.

9. Apparatus according to any one of Claims 1 to 10, **characterised in that** the tube (7) is adapted for passage of an oocyte or embryo.

10. Apparatus according to any one of the preceding claims including a dual-lumen oocyte recovery needle (1), **characterised in that** the tube (20) is connected to a flushing lumen of the needle, that an aspiration tube (7) is connected with the second lumen of the needle, and that a second elongate jacket (30) extends along the length of the aspiration tube to maintain the temperature of an oocyte flowing along the aspiration tube.
